# EUROPEAN PATENT SPECIFICATION

(11) **EP 3 241 199 B1**
(45) Date of publication and mention of the grant of the patent: **26.09.2018**
(21) Application number: 15820959.3
(22) Date of filing: 16.12.2015
(51) Int. Cl.: G08B 21/14, G08B 25/14

(54) **IMAGE BASED SURVEILLANCE SYSTEM**
BILDBASIERTES ÜBERWACHUNGSSYSTEM
SYSTÈME DE SURVEILLANCE SUR LA BASE D'IMAGES

(30) Priority: 29.12.2014 US 201414584027
(43) Date of publication of application: 08.11.2017
(73) Proprietor: Honeywell International Inc., Morris Plains, NJ 07950 (US)
(72) Inventor: AMARAVADI, Vijayapavan, Morris Plains, New Jersey 07950 (US)
(74) Representative: Houghton, Mark Phillip
(86) International application number: PCT/US2015/065991
(87) International publication number: WO 2016/109197

(56) References cited:
- US-A1- 2008 097 731
- US-A1- 2008 133 653
- US-A1- 2014 226 951

## Description

### FIELD

This application relates to safety systems and more particular to hazardous gas detection systems.

### BACKGROUND

Systems are known to protect people and assets from hazardous and combustible gas leaks within areas under surveillance. Such systems are typically based upon the use of a number of gas sensors distributed throughout the area under surveillance.

For example, carbon monoxide detectors may be located near sleeping areas in residences. Similarly, smoke or carbon monoxide detectors may be placed in a kitchen or near a home's heating system.

In an industrial setting involving the use of toxic gases (e.g., hydrogen sulfide, hydrogen dioxide, etc.), one or more gas detectors may be placed near a source and/or a point of consumption of the toxic gas. Gas and/or smoke detectors may also be located throughout the area for the additional protection of people working in the area. Individuals may also wear portable gas detectors which will help in gas detection while moving around the area.

Gas detectors (fixed and portable) within an area are often coupled to a central monitoring panel. In this case, each gas detector may periodically measure a gas level proximate the device and report its readings to the central monitoring panel. The central monitoring system may receive a gas reading from each gas detector and sound a general (or local) alarm if the detected gas exceeds some threshold level. In addition to gas detection, the devices can also communicate about man down, panic situations and compliance dues. Device has an accelerometer to communicate about Man Down situation if the device user does not move for a specified time. Device user him/herself can trigger a Panic situation by pressing a button on the device as needed.

A. display may be used in conjunction with the central monitoring panel. The display may show a map of the area under surveillance and the location of any activated sensors within the area.

While such systems work well, they are often difficult to use. For example, some areas may have hundreds of gas detectors. In such cases, it is difficult for a central monitoring system to reliably receive a reading from each detector and act upon those readings in an expeditious manner. Accordingly, a need exists for better methods of receiving readings from gas detectors and displaying those readings on a central monitoring panel.

US2014/0226951A1 discloses an apparatus and method of using a security system. The method includes the steps of providing a two-dimensional map of geographical features of a secured area on a display, arranging a respective icon for each of a plurality of security sensors on the map, wherein a relative location of the respective icon of each of the plurality of security sensors on the two-dimensional map indicates the relative location of the sensor with respect to each of the geographical features of the map and wherein the respective icon of each of the plurality of sensors that is activated is shown highlighted on the map and providing an adjustable time control device with a time indicator on the map, the time control device causing each of the plurality of icons to enter a highlighted state during each concurrence in time between the activated state of the corresponding sensor and the time indicator.

US 2008/0097731A1 discloses a system and method for graphically displaying information concerning geographically dispersed assets. The method comprises the steps of using a computer program to associate map and/or geographical data with a physical asset; representing each dispersed asset with an icon or other graphical representation on a display; and using an automatic grouping function to combine at least two of the assets into a single icon or other graphical representation of the multiplicity of assets and generate a shape that represents the grouped assets. A corresponding system is also disclosed.

### SUMMARY

The present invention provides a method according to claim 1 of the appended claims.

The invention further provides a system according to claim 10 of the appended claims.

### BRIEF DESCRIPTION OF THE DRAWINGS

FIG. 1 illustrates a block diagram of a safety system in accordance herewith;
FIG. 2 illustrates a screen showing a map with a cluster icon; and
FIG. 3 illustrates a box showing the gas detectors represented by the cluster icon.

### DETAILED DESCRIPTION

It should be understood at the outset that although illustrative implementations of one or more embodiments are illustrated below, the disclosed systems and methods may be implemented using any number of techniques, whether currently known or not yet in existence. The disclosure should in no way be limited to the illustrative implementations, drawings, and techniques illustrated below, but may be modified within the scope of the appended claims along with their full scope of equivalents.

The following brief definition of terms shall apply throughout the application:

The term "comprising" means including but not limited to, and should be interpreted in the manner it is typically used in the patent context;

The phrases "in one embodiment," "according to one embodiment," and the like generally mean that the particular feature, structure, or characteristic following the phrase may be included in at least one embodiment of the present invention, and may be included in more than one embodiment of the present invention (importantly, such phrases do not necessarily refer to the same embodiment);

If the specification describes something as "exemplary" or an "example," it should be understood that refers t to a non-exclusive example;

The terms "about" or approximately" or the like, when used with a number, may mean that specific number, or alternatively, a range in proximity to the specific number, as understood by persons of skill in the art field; and

If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

While disclosed embodiments can take many different forms, specific embodiments thereof are shown in the drawings and will be described herein in detail with the understanding that the present disclosure is to be considered as an exemplification of the principles thereof as well as the best mode of practicing same, and is not intended to limit the application or claims to the specific embodiment illustrated.

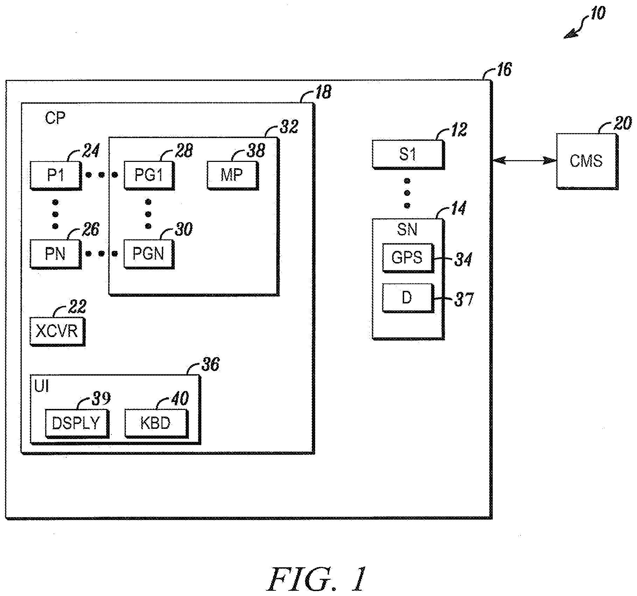

FIG. 1 is a simplified block diagram of a safety system (e.g., a gas monitoring system) 10 shown generally in accordance with an illustrated embodiment. Included within the system is a number of wireless portable detectors (e.g., gas detectors) 12, 14 that detect threats to people and assets within the geographic area 16 under surveillance.

A control panel 18 may monitor each of the detectors for gas levels above a threshold value and for also panic and man-down situations. Upon detecting a gas sensor crossing the threshold value, the control panel may compose and send an alarm message to a central monitoring station 20. The alarm message may include an identifier of the alarm system (e.g., address, account number, etc.), an identifier of the type of alarm and a location of the alarm within the secured area. The central monitoring station may respond by summoning help (e.g., paramedics or other emergency responders, fire department, etc.).

While the control panel is shown within the area under surveillance, it may also be located remotely. Similarly, while the central monitoring station is shown to be remotely located, the central monitoring station may also be located within the area under surveillance.

Included within the control panel and each of the wireless portable gas detectors is a radio frequency transceiver 22. The control panel and each of the wireless detectors may exchange messages under any of a number of different formats (e.g., TDMA, FDMA, etc.).

Also included within the control panel and each of the portable detectors is one or more processor apparatus (processors) 24, 26. Each of the processors may operate under control of one or more computer programs 28, 30 loaded from a non-transitory computer readable medium (memory) 32. As used herein, reference to a step performed by a computer program is also reference to the processor that executed that step.

At least some or all of the portable detectors may also include a position detection device 34. The position detection device may be a GPS device or a triangulating processor that determines the detector's position by reading three or more signal strength values from a set of radio frequency beacons distributed around a periphery of the area under surveillance. A tracking processor within the detector may periodically determine the detector's geographic location and report that location to the control panel.

Similarly, a gas reading processor within each detector may retrieve a gas reading from a respective sensor 37 and compare that reading with a threshold value saved within a memory of the detector. If the reading exceeds the threshold value, then the gas reading processor or a separate reporting processor may send an alarm or high gas reading message to the control panel. The high gas reading message may include an identifier of the detector, the gas reading retrieved from the sensor and an indicator that the gas reading has exceeded the threshold value. The message may also include a recently determined geographic location of the detector at the time of the high gas reading.

Alternatively, the gas reading processor may simply retrieve a gas reading and transmit the gas reading and location of gas reading to the control panel. In this case, the threshold value for each detector may be saved within the memory of the control panel. In response, the alarm is determined within the control panel by comparing the reading with the threshold value.

Included within memory of the control panel may be a map of the area under surveillance. The map may be embodied as a set of geographic features of the area under surveillance (e.g., doors, walls, parking lots, etc.) and a respective geographic coordinate of each feature saved within a file 38.

The safety system may be monitored by a security guard or control room operator via a user interface 36. The user interface 36 may include a display 39 and a keyboard 40.

The security guard or control room operator may monitor the status of the area under surveillance by activating a display processor via the keyboard. The display processor may retrieve the map from the map file and present the map of the display. A location processor may determine a geographic location of each of the portable detectors with respect to the map coordinates and display a respective icon on the map at a corresponding location of the detector.

A status processor may then determine a status of each of the gas detectors and color the respective icons shown in the map. For example, a red icon may indicate an alarmed device or sensor (i.e., the gas read reading exceeds the threshold or device indicates a man down state or panic alarm). An amber icon may indicate a fault condition. A fault condition may indicate that a device calibration is due, that the gas reading does not represent a valid value or that the control panel cannot contact the detector. A green icon indicates that the control panel is in contact with the detector and that the gas reading is in a normal range and does not exceed the threshold value.

In general, the display and status processors may be control room applications whose functionality is accomplished via a number of different safety system programs (e.g., LocaXion Manager (LM) of ConneXt Safety Solutions Pro). The processors allow a guard or other user to perform hazardous gas detection and location identification of field operators (personnel) carrying portable detectors and who are otherwise working in the oil and gas industrial environment. The status processors of conventional safety systems show portable gas detectors on the map as individual moving icons. However, there is no way to represent a group of many closely situated icons if the persons carrying the detectors are working as a group in close proximity. When such persons work in close proximity, this causes the display processor to show the device icons on top one another especially if there are many devices in a particular zone. In this situation it becomes especially difficult to identify one particular device and/or to click on it to show further details. Since the moving devices are shown on a map which is zoomable, it adds to further complication and confusion because the group is not always shown in the same manner while zooming in and out of particular areas of the map image.

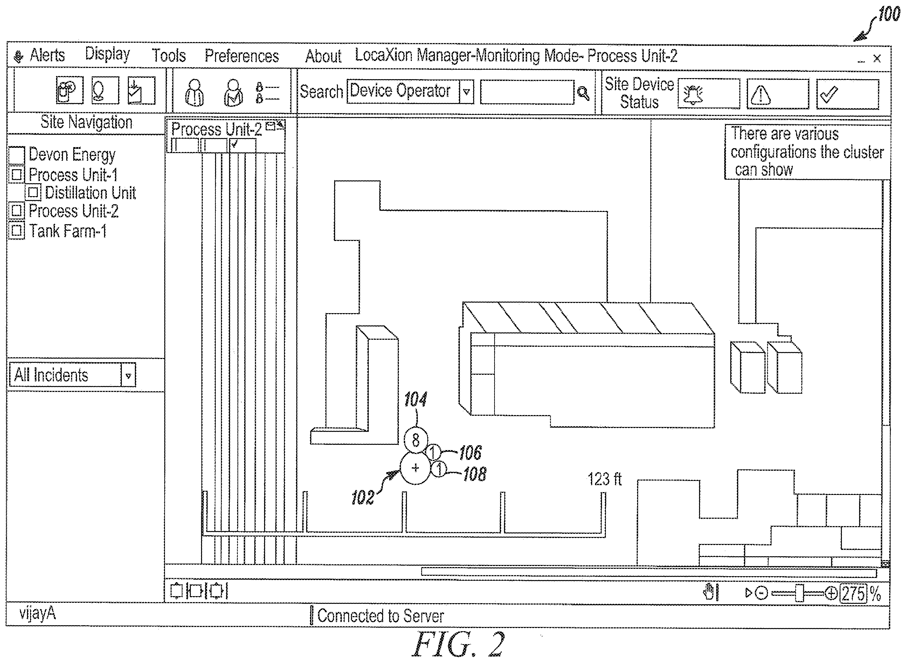

The solution to this problem is to use a group or cluster icon whenever a group of detectors are within a predefined small area. For example, FIG. 2 depicts a screen 100 depicting a map of a monitored area. Included within the map is a group icon 102 that represents a group of portable gas detectors. FIG. 2 shows 10 portable gas detectors (8+1+1). Out of the ten, 8 devices are in alarm, one in fault and another in normal conditions.

As the scale of a given site image/map is known (and also by realizing from the on-screen scenario that it would be difficult to identify a single device at a particular viewable zoom level (before the images becomes pixelated)), it becomes clear that a parameter threshold can be used to control use of the group icon. The use of the threshold is based upon the concept that if five or more devices are close to each other in a particular area (e.g., a 10' by 10' block), then these devices may be shown as a cluster icon. The cluster icon is structured to include details including the number of devices and the status of each device displayed in conjunction with that cluster icon. For example, FIG. 2 depicts the number of alarmed devices represented by the icon shown within a red shaded circle 104 located directly adjacent the icon. In this case, the shaded circle is located directly above the icon and includes a number equal to the number of alarmed gas detectors represented by that group icon. In this case, the group icon represents eight gas detectors and the number 8 is shown within the red shaded circle directly above the group icon.

Similarly, the device with fault status may be represented within another circle 106 directly adjacent and to the upper right of the group icon. In this case, only one of the gas detectors is in fault status and the circle shows the number 1.

The normal status may also displayed within a third circle 108 shown directly adjacent and to the right of the group icon. In this case, only one of the ten gas detectors is in normal state and the circle contains the number 1.

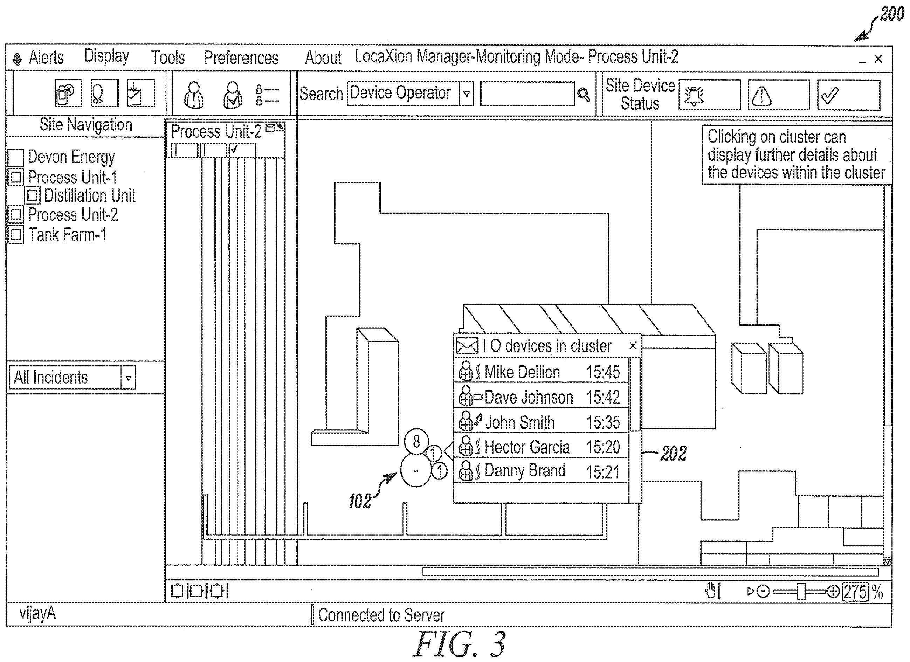

By clicking on the cluster icon, a user can display further details about the devices and their alarms, faults, etc. For example, FIG. 3 depicts a screen 200 where a user has clicked on the cluster icon. In response, a display processor shows a box 202 including partial list of the ten gas detectors and the status of each detector. The user can use the scroll bar on the right of the box to scroll through the remaining gas detectors represented by the cluster icon.

In general, the use of the cluster icon represents only part of the embodiment. Another significant part of the embodiment lies in identifying the correct parameter for controlling the use of the cluster icon based upon the realities of use and its practicality in being able to identify the detectors represented by the cluster icon.

For example, a 3.048 meter by 3.048 meter (10 foot by 10 foot (10' x 10')) block or circle may be used as the parameter for grouping detectors and for replacing the grouped detectors with a single cluster icon. However, a selected parameter of a 10' x 10' block may not be appropriate for all images and users. For example, the parameter may be expanded for use in other situations by other users (e.g., a 9.144 meter x 9.144 meter or 30' x 30' block) or may be otherwise configurable based on the desired image clarity, the site size of the user and the location accuracy the user is seeking.

In general, the locations of the icons (portable detectors) shown on the map of dynamically adjusted based the moving position of each detector. Similarly, the details of the map are also dynamically changed based upon the zoom level. For example, Google maps reveals more data as one is zooming in and reveals less detail as a user zooms out. This is done to prevent the detail from obscuring the map's geographic reference points.

This combination of adjustments based upon both position and zoom level makes it very difficult to reliably read the maps displayed by the display processor. This is because the icons of the detectors at some point also begin to obscure the map's geographic reference points. Because of the balance between the number of detector icons and the map's detail, no standard size or standard for triggering the use of the group pixel can be established for all users. Hence, the use of group pixels based upon the level of map detail renders the concepts described herein different than the detector display methods of conventional security systems and also from conventional screen map visualizations.

In order to use the detector display system of FIG. 1, a user first enters a configuration mode in order to set up the use of the group icon. In the configuration mode, a group icon is selected as a first step in order to set the scale for any given image. Once the scale is set, the user may be asked to provide location accuracy resolution that the user would want the application to support. Based on this resolution, the cluster parameter can be defined for a space from 3.048 meter x 3.048 meter (10' x 10') to 9.144 meter x 9.144 meter (30' x 30') or larger. As the detectors communicate with ommunicate the server about their location, the display processor (or a related processor) begins to group detectors based upon their presence within some predefined area (e.g., 3.048 meter x 3.048 meter or 10' x 10', 4.572 meter x 4.572 meter or 15' x 15' etc.) of the map. If the status processor identifies any group of detects within that predefined area, the processor deletes those detector icons and replaces the deleted icons with a group icon centered over that predefined area. The use of the cluster icon is used irrespective of the zoom percentage imposed by a user on the map.

In general, the system incorporates the steps of a display of a gas detection system depicting a map of a predetermined geographic area and a plurality of portable gas detectors on the map via a respective gas detector icon, a programmed processor of the gas detection system identifying a number of gas detectors of the plurality of portable gas detectors within a predetermined portion of the area depicted on the display, the number exceeding a threshold value and a programmed processor of the gas detection system replacing the icons of the identified gas detectors within the portion with a group icon.

Alternatively, the system includes a gas detection system that uses a plurality of gas detectors to detect hazardous gases within a predetermined geographic area, a display of the gas detection system that depicts a map of the predetermined geographic area and the plurality of portable gas detectors on the map via a respective gas detector icon, a programmed processor of the gas detection system that identifies a number of gas detectors of the plurality of portable gas detectors within a predetermined portion of the area depicted on the display, the number exceeding a threshold value and a programmed processor of the gas detection system that replaces the icons of the identified gas detectors within the portion with a group icon.

Alternatively, the system includes a gas detection system that protects a predetermined geographic area, plurality of portable gas detectors of the gas detection system distributed throughout the area that detect hazardous gases, a display of the gas detection system that depicts a map of the area with the plurality of portable gas detectors displayed on the map via a respective gas detector icon, a programmed processor of the gas detection system that displays a status of each of the plurality of gas detectors on the map via a color indicator, a programmed processor of the gas detection system that identifies a number of gas detectors of the plurality of portable gas detectors within a predetermined portion of the area depicted on the display, the number exceeding a threshold value and a programmed processor of the gas detection system that replaces the icons of the identified gas detectors within the portion with a group icon.

From the foregoing, it will be observed that numerous variations and modifications may be effected without departing from the scope hereof. It is to be understood that no limitation with respect to the specific apparatus illustrated herein is intended or should be inferred. It is, of course, intended to cover by the appended claims all such modifications as fall within the scope of the claims. Further, logic flows depicted in the figures do not require the particular order shown, or sequential order, to achieve desirable results. Other steps may be provided, or steps may be eliminated, from the described flows, and other components may be added to, or removed from the described embodiments.

Embodiments of the disclosure may include methods for indicating alarm and fault status for a plurality of gas detectors. In some embodiments, sensed gas data may be received from a gas detection system, wherein the gas detection system may comprise a plurality of gas detectors. In some embodiments, device information may be received from each of the plurality of gas detectors. In some embodiments, a user interface 36 may display a map of a geographic area, wherein the plurality of gas detectors may be located within the geographic area. In some embodiments, the user interface 36 may indicate the location of a gas detector within the geographic area using a gas detector icon. In some embodiments, the method may comprise identifying when the number of the gas detectors located within a predetermined portion of the geographic area exceeds a threshold value, wherein the predetermined portion may be defined by area size, and wherein the predetermined portion may be located anywhere within the geographical area. In some embodiments, the gas detector icons of the gas detectors located within the predetermined portion may be replaced with a group gas detector icon.

In some embodiments, the method may comprise identifying when the number of gas detectors located within the predetermined portion of the geographic area decreases below the threshold value. In some embodiments, the group gas detector icon may be replaced with individual gas detector icons. In some embodiments, the method may comprise identifying when the number of the gas detectors located within a second predetermined portion of the geographic area exceeds the threshold value, wherein the second predetermined portion of the geographic area may be in a location different from the first predetermined portion of the geographic area. In some embodiments, the gas detector icons of the gas detectors located within the second predetermined portion may be replaced with a group gas detector icon.

In some embodiments, the predetermined portion of the geographic area comprises an area of between approximately 9.290 square meters (100 square feet) and 232.258 square meters (2500 square feet). In some embodiments, the predetermined portion of the geographic area comprises an area of 3.048 meter by 3.048 meter (10 feet by 10 feet). In some embodiments, the predetermined portion of the geographic area comprises an area of 6.096 meter by 6.096 meter (20 feet by 20 feet). In some embodiments, the predetermined portion of the geographic area comprises an area of 7.620 meter by 7.620 meter (25 feet by 25 feet). In some embodiments, the predetermined portion of the geographic area comprises an area of between approximately 2.323 square meters (25 square feet) and 83.613 square meters (900 square feet).

In some embodiments, the threshold value comprises at least five gas detectors. In some embodiments, the threshold value comprises at least ten gas detectors. In some embodiments, the group gas detector icon may be located in the center of the predetermined portion of the geographic area. In some embodiments, the number of gas detectors located within the predetermined portion of the geographic area may be displayed, proximate to the group gas detector icon.

In some embodiments, the received gas data from each of the gas detectors may be compared with one or more threshold values for the gas data. In some embodiments, the number of gas detectors in the group having a detected alarm may be indicated, via the group gas detector icon. In some embodiments, when the group gas detector icon is accessed by a user, the details of the alarm status for the gas detectors in the group may be displayed.

In some embodiments, the received device information from each of the gas detectors may be compared with one or more threshold values. In some embodiments, the number of gas detectors in the group having a detected fault may be indicated, via the group gas detector icon. In some embodiments, when the group gas detector icon is accessed by a user, the details of the fault status for the gas detectors in the group may be displayed.

Embodiments of the disclosure may include a system 100 comprising a plurality of gas detectors 12, 14 operable to communicate sensed gas data and device information, and a control panel 18 comprising a user interface 36 operable to display a map of a geographic area, wherein the plurality of gas detectors 12, 14 are located within the geographic area and indicated by gas detector icons, and a processor 24 operable to receive sensed gas data from the plurality of gas detectors 12, 14; receive device information from the plurality of gas detectors 12, 14; identify when the number of the gas detectors 12, 14 located within a predetermined portion of the geographic area exceeds a threshold value, wherein the predetermined portion is defined by area size, and wherein the predetermined portion can be located anywhere within the geographical area; and replace the gas detector icons of the gas detectors 12, 14 located within the predetermined portion with a group gas detector icon 102.

In some embodiments, the processor 24 may further be operable to identify when the number of gas detectors 12, 14 located within the predetermined portion of the geographic area decreases below the threshold value; and replace the group gas detector icon 102 with individual gas detector icons.

In some embodiments, the processor 24 may further be operable to identify when the number of the gas detectors 12, 14 located within a second predetermined portion of the geographic area exceeds the threshold value, wherein the second predetermined portion of the geographic area is in a location different from the first predetermined portion of the geographic area; replace the gas detector icons of the gas detectors 12, 14 located within the second predetermined portion with a group gas detector icon 102.

In some embodiments, the user interface 36 may be further operable to display, proximate to the group gas detector icon 102, the number of gas detectors 12, 14 located within the predetermined portion of the geographic area. In some embodiments, the processor 24 may be further operable to compare the received gas data from each of the gas detectors 12, 14 with one or more threshold values for the gas data; and indicate, via the group gas detector icon 102, the number of gas detectors 12, 14 in the group having a detected alarm, and wherein the user interface 36 displays, when the group gas detector icon 102 is accessed by a user, the details of the alarm status for the gas detectors 12, 14 in the group.

In some embodiments, the processor 24 may be further operable to compare the received device information from each of the gas detectors 12, 14 with one or more threshold values; and indicate, via the group gas detector icon 102, the number of gas detectors 12, 14 in the group having a detected fault, and wherein the user interface 36 displays, when the group gas detector icon 102 is accessed by a user, the details of the fault status for the gas detectors 12, 14 in the group.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the claims. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present invention(s). Furthermore, any advantages and features described above may relate to specific embodiments, but shall not limit the application of such issued claims to processes and structures accomplishing any or all of the above advantages or having any or all of the above features.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure. Specifically and by way of example, although the headings might refer to a "Field," the claims should not be limited by the language chosen under this heading to describe the so-called field. Further, a description of a technology in the "Background" is not to be construed as an admission that certain technology is prior art to any invention(s) in this disclosure. Neither is the "Summary" to be considered as a limiting characterization of the invention(s) set forth in issued claims. Furthermore, any reference in this disclosure to "invention" in the singular should not be used to argue that there is only a single point of novelty in this disclosure. Multiple inventions may be set forth according to the limitations of the multiple claims issuing from this disclosure, and such claims accordingly define the invention(s), and their equivalents, that are protected thereby. In all instances, the scope of the claims shall be considered on their own merits in light of this disclosure, but should not be constrained by the headings set forth herein.

Use of broader terms such as comprises, includes, and having should be understood to provide support for narrower terms such as consisting of, consisting essentially of, and comprised substantially of. Use of the term "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

While several embodiments have been provided in the present disclosure, it should be understood that the disclosed systems and methods may be embodied in many other specific forms without departing from the scope of the present disclosure. The present examples are to be considered as illustrative and not restrictive, and the intention is not to be limited to the details given herein. For example, the various elements or components may be combined or integrated in another system or certain features may be omitted or not implemented.

Also, techniques, systems, subsystems, and methods described and illustrated in the various embodiments as discrete or separate may be combined or integrated with other systems, modules, techniques, or methods without departing from the scope of the present claims. Other items shown or discussed as directly coupled or communicating with each other may be indirectly coupled or communicating through some interface, device, or intermediate component, whether electrically, mechanically, or otherwise. Other examples of changes, substitutions, and alterations are ascertainable by one skilled in the art and could be made without departing from the scope disclosed herein.

## Claims

1. A method for indicating status for a plurality of gas detectors (12, 14) comprising:
receiving sensed gas data, device information, and a geographic location from each of the plurality of gas detectors (12, 14);
displaying, on a display (39) of a user interface (36), i) a map of a geographic area and ii) a plurality of gas detector icons on the map, wherein the plurality of gas detector icons correspond to the geographic location for each of the plurality of gas detectors (12, 14);
identifying a number of the gas detectors (12, 14) of the plurality of gas detectors (12, 14) which are located within a predetermined portion of the geographic area on the display (39); and
based on the number of the gas detectors which exceeds a threshold value within the predetermined portion of the area, replacing, on the display (39), the gas detector icons of the number of gas detectors (12, 14) identified within the predetermined portion of the area with a group gas detector icon (102) and an alarm status icon directly (104) adjacent the group gas detector icon (102),
wherein the group gas detector icon (102) indicates a numerical value for the number of gas detectors (12, 14) in the group, and
wherein the alarm status icon (104) indicates a numerical value for the number of gas detectors (12, 14) in the group which are in alarm.

2. The method of claim 1, further comprising:
identifying when the number of the gas detectors (12, 14) located within the predetermined portion of the geographic area decreases below the threshold value; and
replacing the group gas detector icon (102) with individual gas detector icons.

3. The method of claim 1, wherein the predetermined portion is defined by area size, and wherein the predetermined portion can be located anywhere within the geographical area.

4. The method of claim 1, wherein the predetermined portion of the geographic area comprises an area of between approximately 9.290 and 232.258 square meters.

5. The method of claim 1, wherein the threshold value comprises at least five gas detectors (12, 14).

6. The method of claim 1, wherein the group gas detector icon (102) is located in the center of the predetermined portion of the geographic area.

7. The method of claim 1, further comprising displaying a normal status icon (108) directly adjacent to the group gas detector icon (102), wherein the normal status icon (108) indicates a numerical value for the number of gas detectors (12, 14) identified within the predetermined portion of the geographic area which are in a normal state.

8. The method of claim 1, further comprising:
comparing the received gas data from each of the plurality of gas detectors (12, 14) with a second threshold value for the gas data;
determining the gas data for at least one of the plurality of gas detectors (12, 14) exceeds the second threshold value; and
displaying the alarm status icon (104) above the group gas detector icon (102).

9. The method of claim 1, further comprising:
comparing the received device information from each of the plurality of gas detectors (12, 14) with a second threshold value;
determining a fault condition exists for at least one of the number of gas detectors identified; and
displaying a fault status icon (106) directly adjacent the group gas detector icon (102), wherein the fault status icon (106) indicates a numerical value for the number of gas detectors (12, 14) identified which have the fault condition.

10. A system comprising:
a plurality of gas detectors (12, 14); and
a control panel (18),
wherein each of the plurality of gas detectors (12, 14) is configured to communicate sensed gas data, device information, and a geographic location to the control panel (18),
wherein the control panel (18) comprises a display (39) of a user interface (36) configured to display a map of a geographic area, wherein the plurality of gas detectors (12, 14) are located within the geographic area and are indicated by gas detector icons, and a processor (24) configured to:
receive the sensed gas data, the device information, and the geographic location from each of the plurality of gas detectors (12, 14);
identify a number of the plurality of gas detectors (12, 14) located within a predetermined portion of the geographic area that exceeds a threshold value,; and
based on the number of gas detectors that exceeds the threshold value within the predetermined portion of the geographic area, replace, on the display (39), the gas detector icons of the number of gas detectors (12, 14) located within the predetermined portion with a group gas detector icon (102) and an alarm status icon directly (104) adjacent the group gas detector icon (102),
wherein the group gas detector icon (102) indicates a numerical value for the number of gas detectors (12, 14) in the group, and
wherein the alarm status icon (104) indicates a numerical value for the number of gas detectors (12, 14) in the group which are in alarm.

11. The system of claim 10, wherein the processor (24) is further configured to:
identify when the number of gas detectors (12, 14) located within the predetermined portion of the geographic area decreases below the threshold value; and
replace the group gas detector icon (102) with individual gas detector icons.

12. The system of claim 11, wherein the predetermined portion is defined by area size, and wherein the predetermined portion can be located anywhere within the geographical area.

13. The system of claim 11, wherein the display (39) of the user interface (36) is further configured to display, directly adjacent to the group gas detector icon (102), a normal status icon (108) which indicates a numerical value for the number of gas detectors (12, 14) identified which are in a normal state.

14. The system of claim 11, wherein the processor (24) is further configured to:
compare the sensed gas data received from each of the plurality of gas detectors (12, 14) with a second threshold value for the gas data; and
determine the gas data for at least one of the plurality of gas detectors (12, 14) exceeds the second threshold value,
wherein the display (39) of the user interface (36) displays the alarm status icon (104) above the group gas detector icon (102).

15. The system of claim 11, wherein the processor (24) is further configured to:
compare the device information received from each of the gas detectors (12, 14) with a second threshold value; and
determine a fault condition exists for at least one of the number of gas detectors (12, 14) identified,
wherein the display (39) of the user interface (36) displays a fault status icon (106) directly adjacent the group gas detector icon (102), wherein the fault status icon (106) indicates a numerical value for the number of gas detectors (12, 14) identified which have the fault condition.

## Patentansprüche

1. Verfahren, um den Status für mehrere Gaserkennungseinrichtungen (12, 14) anzuzeigen, das Folgendes umfasst:
Empfangen von erfassten Gasdaten, Einrichtungsinformationen und geographischen Orten von den mehreren Gaserkennungseinrichtungen (12, 14);
Anzeigen auf einer Anzeigevorrichtung (39) von einer Anwenderschnittstelle (36) i) einer Karte eines geographischen Bereichs und ii) mehrerer Gaserkennungseinrichtungssymbole auf der Karte, wobei die mehreren Gaserkennungseinrichtungssymbole den geographischen Orten der mehreren Gaserkennungseinrichtungen (12, 14) entsprechen;
Erkennen mehrerer Gaserkennungseinrichtungen (12, 14) der mehreren Gaserkennungseinrichtungen (12, 14), die sich in einem vorgegebenen Abschnitt des geographischen Bereichs auf der Anzeigevorrichtung (39) befinden; und
auf der Grundlage der Anzahl der Gaserkennungseinrichtungen, die im vorgegebenen Abschnitt des Bereichs einen Schwellenwert überschreitet, Ersetzen auf der Anzeigevorrichtung (39) der Gaserkennungseinrichtungssymbole von den mehreren Gaserkennungseinrichtungen (12, 14), die im vorgegebenen Abschnitt des Bereichs erkannt wurden, durch ein Gruppengaserkennungseinrichtungssymbol (102) und ein Alarmzustandssymbol (104), das dem Gruppengaserkennungseinrichtungssymbol (102) direkt benachbart ist, wobei
das Gruppengaserkennungseinrichtungssymbol (102) einen Zahlenwert für die Anzahl von Gaserkennungseinrichtungen (12, 14) in der Gruppe anzeigt und
das Alarmzustandssymbol (104) einen Zahlenwert für die Anzahl von Gaserkennungseinrichtungen (12, 14) in der Gruppe, die sich im Alarmzustand befinden, anzeigt.

2. Verfahren nach Anspruch 1, das ferner Folgendes umfasst:
Erkennen, wann die Anzahl der Gaserkennungseinrichtungen (12, 14), die sich im vorgegebenen Abschnitt des geographischen Bereichs befinden, unter den Schwellenwert abnimmt; und
Ersetzen des Gruppengaserkennungseinrichtungssymbols (102) durch individuelle Gaserkennungseinrichtungssymbole.

3. Verfahren nach Anspruch 1, wobei der vorgegebene Abschnitt durch eine Bereichsgröße definiert ist und der vorgegebene Abschnitt sich überall im geographischen Bereich befinden kann.

4. Verfahren nach Anspruch 1, wobei der vorgegebene Abschnitt des geographischen Bereichs einen Bereich von etwa 9,290 bis 232,258 Quadratmetern umfasst.

5. Verfahren nach Anspruch 1, wobei der Schwellenwert mindestens fünf Gaserkennungseinrichtungen (12, 14) umfasst.

6. Verfahren nach Anspruch 1, wobei sich das Gruppengaserkennungseinrichtungssymbol (102) im Zentrum des vorgegebenen Abschnitts des geographischen Bereichs befindet.

7. Verfahren nach Anspruch 1, das ferner das Anzeigen eines Normalzustandssymbols (108), das dem Gruppengaserkennungseinrichtungssymbol (102) direkt benachbart ist, umfasst, wobei das Normalzustandssymbol (108) einen Zahlenwert für die Anzahl von Gaserkennungseinrichtungen (12, 14), die im vorgegebenen Abschnitt des geographischen Bereichs erkannt wurden und die sich in einem Normalzustand befinden, anzeigt.

8. Verfahren nach Anspruch 1, das ferner Folgendes umfasst:
Vergleichen der von jeder der mehreren Gaserkennungseinrichtungen (12, 14) empfangenen Gasdaten mit einem zweiten Schwellenwert für die Gasdaten;
Bestimmen, dass die Gasdaten für mindestens eine der mehreren Gaserkennungseinrichtungen (12, 14) den zweiten Schwellenwert überschreiten; und
Anzeigen des Alarmzustandssymbols (104) über dem Gruppengaserkennungseinrichtungssymbol (102).

9. Verfahren nach Anspruch 1, das ferner Folgendes umfasst:
Vergleichen der von jeder der mehreren Gaserkennungseinrichtungen (12, 14) empfangenen Einrichtungsinformationen mit einem zweiten Schwellenwert;
Bestimmen, dass ein Fehlerzustand für mindestens eine der mehreren erkannten Gaserkennungseinrichtungen vorliegt; und
Anzeigen eines Fehlerzustandssymbols (106), das dem Gruppengaserkennungseinrichtungssymbol (102) direkt benachbart ist, wobei das Fehlerzustandssymbol (106) einen Zahlenwert für die Anzahl von Gaserkennungseinrichtungen (12, 14), die erkannt wurden und die den Fehlerzustand aufweisen, anzeigt.

10. System, das Folgendes umfasst:
mehrere Gaserkennungseinrichtungen (12, 14); und
eine Steuertafel (18), wobei
jede der mehreren Gaserkennungseinrichtungen (12, 14) konfiguriert ist, erfasste Gasdaten, Einrichtungsinformationen und geographische Orte zu der Steuertafel (18) zu kommunizieren,
die Steuertafel (18) eine Anzeigevorrichtung (39) von einer Anwenderschnittstelle (36) umfasst, die konfiguriert ist, eine Karte von einem geographischen Bereich anzuzeigen, wobei sich die mehreren Gaserkennungseinrichtungen (12, 14) im geographischen Bereich befinden und durch Gaserkennungssymbole angezeigt werden, und ein Prozessor (24) konfiguriert ist,
die erfassten Gasdaten, die Einrichtungsinformationen und die geographischen Orte von den mehreren Gaserkennungseinrichtungen (12, 14) zu empfangen;
Erkennen einer Anzahl der mehreren Gaserkennungseinrichtungen (12, 14), die sich in einem vorgegebenen Abschnitt des geographischen Bereichs befinden, die einen Schwellenwert überschreitet; und
auf der Grundlage der Anzahl der Gaserkennungseinrichtungen, die im vorgegebenen Abschnitt des geographischen Bereichs den Schwellenwert überschreitet, Ersetzen auf der Anzeigevorrichtung (39) der Gaserkennungseinrichtungssymbole von den mehreren Gaserkennungseinrichtungen (12, 14), die sich im vorgegebenen Abschnitt befinden, durch ein Gruppengaserkennungseinrichtungssymbol (102) und ein Alarmzustandssymbol (104), das dem Gruppengaserkennungseinrichtungssymbol (102) direkt benachbart ist, wobei
das Gruppengaserkennungseinrichtungssymbol (102) einen Zahlenwert für die Anzahl von Gaserkennungseinrichtungen (12, 14) in der Gruppe anzeigt und
das Alarmzustandssymbol (104) einen Zahlenwert für die Anzahl von Gaserkennungseinrichtungen (12, 14) in der Gruppe, die sich im Alarmzustand befinden, anzeigt.

11. System nach Anspruch 10, wobei der Prozessor (24) ferner konfiguriert ist zum
Erkennen, wann die Anzahl von Gaserkennungseinrichtungen (12, 14), die sich im vorgegebenen Abschnitt des geographischen Bereichs befinden, unter den Schwellenwert abnimmt; und
Ersetzen des Gruppengaserkennungseinrichtungssymbols (102) durch individuelle Gaserkennungseinrichtungssymbole.

12. System nach Anspruch 11, wobei der vorgegebene Abschnitt durch eine Bereichsgröße definiert ist und der vorgegebene Abschnitt sich überall im geographischen Bereich befinden kann.

13. System nach Anspruch 11, wobei die Anzeigevorrichtung (39) der Anwenderschnittstelle (36) ferner konfiguriert ist, direkt benachbart zum Gruppengaserkennungseinrichtungssymbol (102) ein Normalzustandssymbol (108) anzuzeigen, das einen Zahlenwert für die Anzahl von Gaserkennungseinrichtungen (12, 14), die sich in einem Normalzustand befinden, anzeigt.

14. System nach Anspruch 11, wobei der Prozessor (24) ferner konfiguriert ist zum
Vergleichen der von jeder der mehreren Gaserkennungseinrichtungen (12, 14) empfangenen Gasdaten mit einem zweiten Schwellenwert für die Gasdaten; und
Bestimmen, dass die Gasdaten für mindestens eine der mehreren Gaserkennungseinrichtungen (12, 14) den zweiten Schwellenwert überschreiten, wobei
die Anzeigevorrichtung (39) der Anwenderschnittstelle (36) das Alarmzustandssymbol (104) über dem Gruppengaserkennungseinrichtungssymbol (102) anzeigt.

15. System nach Anspruch 11, wobei der Prozessor (24) ferner konfiguriert ist zum
Vergleichen der von jeder der mehreren Gaserkennungseinrichtungen (12, 14) empfangenen Einrichtungsinformationen mit einem zweiten Schwellenwert; und
Bestimmen, dass ein Fehlerzustand für mindestens eine der mehreren erkannten Gaserkennungseinrichtungen (12, 14) vorliegt, wobei
die Anzeigevorrichtung (39) der Anwenderschnittstelle (36) ein Fehlerzustandssymbol (106), das dem Gruppengaserkennungseinrichtungssymbol (102) direkt benachbart ist, anzeigt, wobei das Fehlerzustandssymbol (106) einen Zahlenwert für die Anzahl von Gaserkennungseinrichtungen (12, 14), die erkannt wurden und die den Fehlerzustand aufweisen, anzeigt.

## Revendications

1. Procédé pour indiquer l'état d'une pluralité de détecteurs de gaz (12, 14) comprenant les étapes suivantes :
recevoir des données de gaz détectées, des informations de dispositif et un emplacement géographique à partir de chacun de la pluralité de détecteurs de gaz (12, 14) ;
afficher, sur un écran d'affichage (39) d'une interface utilisateur (36), i) une carte d'une zone géographique et ii) une pluralité d'icônes de détecteurs de gaz sur la carte, où la pluralité d'icônes de détecteurs de gaz correspond à l'emplacement géographique pour chacun de la pluralité des détecteurs de gaz (12, 14) ;
identifier un certain nombre de détecteurs de gaz (12, 14) de la pluralité des détecteurs de gaz (12, 14) qui sont situés dans une partie prédéterminée de la zone géographique sur l'écran d'affichage (39) ; et
sur la base du nombre de détecteurs de gaz qui dépasse une valeur seuil dans la partie prédéterminée de la zone, remplacer, sur l'écran d'affichage (39), les icônes de détecteurs de gaz des détecteurs de gaz (12, 14) identifiés dans la partie prédéterminée de la zone par une icône de détecteur de gaz de groupe (102) et
une icône d'état d'alarme (104) directement adjacente à l'icône de détecteur de gaz de groupe (102),
où l'icône de détecteur de gaz de groupe (102) indique une valeur numérique pour le nombre de détecteurs de gaz (12, 14) dans le groupe, et
où l'icône d'état d'alarme (104) indique une valeur numérique pour le nombre de détecteurs de gaz (12, 14) du groupe qui sont en alarme.

2. Procédé selon la revendication 1, comprenant en outre les étapes suivantes :
identifier quand le nombre de détecteurs de gaz (12, 14) situés dans la partie prédéterminée de la zone géographique diminue en dessous de la valeur seuil ; et
remplacer l'icône de détecteur de gaz de groupe (102) par des icônes de détecteurs de gaz individuelles.

3. Procédé selon la revendication 1, dans lequel la partie prédéterminée est définie par la taille de la zone, et où la partie prédéterminée peut être située n'importe où dans la zone géographique.

4. Procédé selon la revendication 1, dans lequel la partie prédéterminée de la zone géographique comprend une zone comprise entre environ 9,290 et 232,258 mètres carrés.

5. Procédé selon la revendication 1, dans lequel la valeur seuil comprend au moins cinq détecteurs de gaz (12, 14).

6. Procédé selon la revendication 1, dans lequel l'icône de détecteur de gaz de groupe (102) est située au centre de la partie prédéterminée de la zone géographique.

7. Procédé selon la revendication 1, comprenant en outre d'afficher une icône d'état normal (108) de manière directement adjacente à l'icône de détecteur de gaz de groupe (102), où l'icône d'état normal (108) indique une valeur numérique pour le nombre de détecteurs de gaz (12, 14) identifiés dans la partie prédéterminée de la zone géographique qui sont dans un état normal.

8. Procédé selon la revendication 1, comprenant en outre les étapes suivantes :
comparer les données de gaz reçues de chacun de la pluralité des détecteurs de gaz (12, 14) à une seconde valeur seuil pour les données de gaz ;
déterminer les données de gaz pour au moins l'un de la pluralité des détecteurs de gaz (12, 14) qui dépassent la seconde valeur seuil ; et
afficher l'icône d'état d'alarme (104) au-dessus de l'icône de détecteur de gaz de groupe (102).

9. Procédé selon la revendication 1, comprenant en outre les étapes suivantes :
comparer les informations de dispositif reçues de chacun de la pluralité des détecteurs de gaz (12, 14) à une seconde valeur seuil ;
déterminer qu'une condition de défaillance existe pour au moins un des détecteurs de gaz identifiés ; et
afficher une icône d'état de défaillance (106) de manière directement adjacente à l'icône de détecteur de gaz de groupe (102), où l'icône d'état de défaillance (106) indique une valeur numérique pour le nombre de détecteurs de gaz (12, 14) identifiés qui présentent l'état de défaillance.

10. Système comprenant :
une pluralité de détecteurs de gaz (12, 14) ; et
un panneau de commande (18),
où chacun de la pluralité de détecteurs de gaz (12, 14) est configuré pour communiquer des données de gaz détectées, des informations de dispositif et un emplacement géographique au panneau de commande (18),
où le panneau de commande (18) comprend un écran d'affichage (39) d'une interface utilisateur (36) configuré pour afficher une carte d'une zone géographique, où la pluralité de détecteurs de gaz (12, 14) sont situés dans la zone géographique et sont indiqués par des icônes de détecteurs de gaz, et un processeur (24) configuré pour :
recevoir les données de gaz détectées, les informations de dispositif et l'emplacement géographique de chacun de la pluralité des détecteurs de gaz (12, 14) ;
identifier un certain nombre de la pluralité des détecteurs de gaz (12, 14) qui sont situés dans une partie prédéterminée de la zone géographique qui dépasse une valeur seuil ; et
sur la base du nombre de détecteurs de gaz qui dépasse la valeur seuil dans la partie prédéterminée de la zone géographique, remplacer, sur l'écran d'affichage (39),
les icônes de détecteurs de gaz des détecteurs de gaz (12, 14) situés dans la partie prédéterminée par une icône de détecteur de gaz de groupe (102) et une icône d'état d'alarme (104) directement adjacente à l'icône de détecteur de gaz de groupe (102),
où l'icône de détecteur de gaz de groupe (102) indique une valeur numérique pour le nombre de détecteurs de gaz (12, 14) dans le groupe, et
où l'icône d'état d'alarme (104) indique une valeur numérique pour le nombre de détecteurs de gaz (12, 14) du groupe qui sont en alarme.

11. Système selon la revendication 10, dans lequel le processeur (24) est en outre configuré pour :
identifier quand le nombre de détecteurs de gaz (12, 14) situés dans la partie prédéterminée de la zone géographique diminue en dessous de la valeur seuil ; et
remplacer l'icône de détecteur de gaz de groupe (102) par des icônes de détecteurs de gaz individuelles.

12. Système selon la revendication 11, dans lequel la partie prédéterminée est définie par la taille de la zone, et où la partie prédéterminée peut être située n'importe où dans la zone géographique.

13. Système selon la revendication 11, dans lequel l'écran d'affichage (39) de l'interface utilisateur (36) est en outre configuré pour afficher, de manière directement adjacente à l'icône de détecteur de gaz de groupe (102), une icône d'état normal (108) qui indique une valeur numérique pour le nombre de détecteurs de gaz (12, 14) identifiés qui sont dans un état normal.

14. Système selon la revendication 11, dans lequel le processeur (24) est en outre configuré pour :
comparer les données de gaz détectées reçues de chacun de la pluralité des détecteurs de gaz (12, 14) à une seconde valeur seuil pour les données de gaz ; et
déterminer les données de gaz pour au moins l'un de la pluralité des détecteurs de gaz (12, 14) qui dépassent la seconde valeur seuil,
où l'affichage (39) de l'interface utilisateur (36) affiche l'icône d'état d'alarme (104) au-dessus de l'icône de détecteur de gaz de groupe (102).

15. Système selon la revendication 11, dans lequel le processeur (24) est en outre configuré pour :
comparer les informations de dispositif reçues de chacun des détecteurs de gaz (12, 14) à une seconde valeur seuil ;
déterminer qu'une condition de défaillance existe pour au moins un des détecteurs de gaz (12, 14) identifiés ; et
où l'écran d'affichage (39) de l'interface utilisateur (36) affiche une icône d'état de défaillance (106) de manière directement adjacente à l'icône de détecteur de gaz de groupe (102), où l'icône d'état de défaillance (106) indique une valeur numérique pour le nombre de détecteurs de gaz (12, 14) identifiés qui présentent l'état de défaillance.
